# EUROPEAN PATENT SPECIFICATION

(11) **EP 2 853 276 B1**
(45) Date of publication and mention of the grant of the patent: **04.05.2016**
(21) Application number: 14177153.5
(22) Date of filing: 15.07.2014
(51) Int. Cl.: A61L 26/00

(54) **Composition of Scar Gel**
Zusammensetzung von Narbengewebegel
Composition de gel cicatrisant

(30) Priority: 25.09.2013 TW 102134580
(43) Date of publication of application: 01.04.2015
(73) Proprietor: Far Eastern New Century Corporation, Taipei (TW)
(72) Inventor: Wu, Yong-Yi, Taoyuan County 320 (TW); Huang, Ching-Ping, Taoyuan County 320 (TW); Shih, Ai-Yun, Taoyuan County 320 (TW)
(74) Representative: D Young & Co LLP

(56) References cited:
- WO-A1-03/009877
- GB-A- 2 407 496

## Description

### BACKGROUND

### Field of Invention

The present disclosure relates to a composition. More particularly, the present disclosure relates to a composition of scar gel.

### Description of Related Art

Scar is a normal connective fibrous tissue, which is formed for repairing trauma, inflammation, burns and other wounds. However, in some cases, scar may be formed in depressive progresses and affect the appearance. For example, prominent fat thick, redness or pigmentation problems may occur during scar formation. Some scars can even be turned into hypertrophic scar or keloid, which could seriously affect the appearance.

For this type of abnormal scars, clinical trials have confirmed that silicone gel is significant effective for treating those abnormal scars. Moisture loss through the skin caused by new epithelial tissue, which is not normalized yet, can be controlled by the silicone gel applied on the wound. Therefore, abnormal proliferation of fibroblasts is effectively suppressed without producing excess collagen and significant uplift scars. As US patents (US8021683, US5741509) disclosed, the compositions of those scar gels include a silicone fluid with thousands to ten thousands cps viscosity (cP) for providing moisture to scars; a volatile diluent with low viscosity for the use of quick-drying; and a fumed silica as a thickener to the scar gel for easy application. However, the fumed silica, as the thickener in those scar gels, does not offer long-lasting homogeneous keeping effect, such that delamination in those scar gels frequently occurred and transparent and low viscosity volatile diluent will first be extruded rather than a uniform phase of scar gel. It results in poor use of perception. In addition, the fumed silica has to be produced in complicated processes, and therefore requires higher production costs. Furthermore, white powder precipitation is still a problem of using those scar gels, and affects the appearance while applying those scar gels. Additional examples include WO 03/009877 A1 and GB2407496 A.

In this regard, developing a composition of scar gel with long-lasting homogeneous keeping, embellishing to cover white powder precipitation, and lower costing is still a focus in industry of medical cosmetology.

### SUMMARY

The present disclosure provides a composition of scar gel. The composition of scar gel includes a precipitated silica powder instead of the fumed silica as the thickener to the scar gel. Therefore, the cost of the composition of scar gel is reduced, and the precipitated silica powder offers long-lasting homogeneous keeping effect for better use of perception. In addition, the composition of scar gel also includes an embellishing powder to adjust color of scar gel for better appearance while the scar gel is applied.

The present disclosure relates to a composition of scar gel. The composition of scar gel includes a silicone fluid, a volatile diluent, a precipitated silica powder, and an embellishing powder. The silicone fluid is fully miscible with the volatile diluent, and the precipitated silica powder and the embellishing powder are uniformly dispersed in the silicone fluid and the volatile diluent.

In various embodiments of the present disclosure, the embellishing powder includes iron oxide, manganese oxide, chromium oxide, or combinations thereof.

In various embodiments of the present disclosure, a weight percentage of the silicone fluid is substantially 60∼74%, a weight percentage of the volatile diluent is substantially 16∼28%, a weight percentage of the precipitated silica powder is substantially 3∼23%, and a weight percentage of the embellishing powder is substantially 0.1∼2%.

In various embodiments of the present disclosure, a pH value of the precipitated silica powder is substantially 6∼8.

In various embodiments of the present disclosure, the composition of scar gel further includes a light-damage protection component, and a weight percentage of the light-damage protection component is substantially 0.1∼10%.

In various embodiments of the present disclosure, the light-damage protection component comprises a physical light-damage protection component, a chemical light-damage protection component, or a combination thereof.

In various embodiments of the present disclosure, the physical light-damage protection component comprises titanium dioxide, zinc oxide, zirconium oxide, and the chemical light-damage protection component comprises salicylic acid, avobezone, benzoate-4, cinoxate, dioxybenzone, homosalate, mexoryl SX, methyl anthranilate, octocrylene, oxy-molybdate-40, oxybezone, octyl salicylate, octyl methoxycinnamate, octyl dimethy PABA, padimate-O, para amino benzoic acid, pheny benzimidazole sulfonic acid, roxadimate, sulisobenzone, trolamine salicylate.

In various embodiments of the present disclosure, the composition of scar gel further includes an inorganic salts powder, and a weight percentage of the inorganic salts powder is substantially 0.1∼5%.

In various embodiments of the present disclosure, the inorganic salts powder comprises barium sulfate, strontium sulfate, magnesium sulfate, ferric hydroxide, calcium hydroxide, sodium chloride, potassium chloride, ammonium chloride, monoethanolamine, diethanolamine chloride, sodium sulfate, sodium phosphate, disodium hydrogen phosphate, sodium tripolyphosphate, magnesium silicate, silicon dioxide, sodium magnesium silicate, sodium silicate, hydrated silica, montmorillonite, lithium magnesium sodium silicate, hectorite, stearyl ammonium montmorillonite, stearyl ammonium hectorite, quaternary ammonium montmorillonite 90, quaternary ammonium montmorillonite 18, quaternary ammonium hectorite 18, or combinations thereof.

In various embodiments of the present disclosure, the silicone fluid comprises a mixture of polydimethylsiloxane and dimethylsiloxane, and the volatile diluent comprises linear or cyclic volatile siloxane.

It is to be understood that both the foregoing general description and the following detailed description are by examples, and are intended to provide further explanation of the disclosure as claimed.

### DETAILED DESCRIPTION

Reference will now be made in detail to the present embodiments of the disclosure, examples of which are illustrated in the accompanying drawings. Wherever possible, the same reference numbers are used in the drawings and the description to refer to the same or like parts.

The composition of scar gel in the present disclosure includes a silicone fluid, a volatile diluent, a precipitated silica powder, and an embellishing powder. The silicone fluid could be also called as a polymer organic silicon compound. The silicone fluid has various advantages, such as being colorless, inert, non-toxic, well occlusive, and having low moisture permeability. Therefore, the silicone fluid is widely applied in various products of cosmetic dermatology. In various embodiments of the present disclosure, the silicone fluid includes a mixture of polysiloxane and siloxane. The mixture of polysiloxane and siloxane could be prepared in any proper ratio of polysiloxane and siloxane according to actual requirements corresponding to products of cosmetic dermatology. For example, since polysiloxane has different molecular weight and viscosity from those of siloxane, a proper ratio of polysiloxane and siloxane in the mixture could be prepared for a product of cosmetic dermatology with a specific viscosity. The polysiloxane, for example, could be cyclic methyl siloxane, poly methyl phenyl siloxane, poly dimethyl siloxane, bis-octadecyloxy dimethyl polysiloxane, polyether siloxane copolymer, poly dimethylsiloxane, or derivatives thereof. The polysiloxane increases lubricity of products of cosmetic dermatology. When products of cosmetic dermatology containing the polysiloxane are applied on skin, a waterproof layer could be formed uniformly thereon without stickness. In various embodiments of the present disclosure, the silicone fluid includes a mixture of polydimethylsiloxane (PDMS) and dimethylsiloxane. The PDMS in liquid phase is a viscous liquid, and could be also called in silicone oil. The PDMS could be regarded as a mixture of chain-shaped polydimethylsiloxanes with various degree of polymerization. All of side groups and terminal groups in the chain-shaped polydimethylsiloxanes are hydrocarbyl groups such as methyl, ethyl, or phenyl. The silicone oil is a colorless, odorless, non-toxic, and non-volatile liquid.

The volatile diluent is siloxane or its derivative, which has relatively low molecular weight, such that the volatile diluent is volatile and has low viscosity. Therefore, the volatile diluent offers convenience of quick-drying when the scar gel of the present disclosure is applied on skin. The volatile diluent, for example, could includes cyclic methyl siloxane, poly methyl phenyl siloxane, poly dimethyl siloxane, bis-octadecyloxy dimethyl polysiloxane, polyether siloxane copolymer, poly dimethylsiloxane, or derivatives thereof, which has relatively low molecular weight and is volatile. In various embodiments of the present disclosure, the volatile diluent includes linear or cyclic volatile siloxane. In summary, among the composition of scar gel according to the present disclosure, siloxane monomers and polymers of the silicone fluid could be the same with, or different from those of the volatile diluent. The silicone fluid could be a mixture of polydimethylsiloxanes with various degree of polymerization, and the silicone fluid has relatively high molecular weight. Therefore, the silicone fluid is non-volatile and provides high occlusive dressing and low moisture permeability to assist healing of the scar attached. On the other hand, the volatile diluent could be volatile linear or cyclic volatile siloxane, and has relatively low molecular weight. Therefore, the volatile diluent has low viscosity and is volatile to provide convenience of quick-drying while applying the scar gel of the present disclosure. Since both of the silicone fluid and the volatile diluent include siloxane, the silicone fluid and the volatile diluent are fully miscible, and therefore constitute a main part in the composition of scar gel of the present disclosure.

It should be noticed that the composition of scar gel of the present disclosure includes the precipitated silica powder. The precipitated silica powder thickens aforementioned mixture of the silicone fluid and the volatile diluent, and consequently a gel is formed. Therefore, users could easily apply the gel on their skin without stickiness. As aforementioned, the fumed silica, as the thickener of the gel, has to be produced in complicated processes. Therefore, the cost of producing the fumed silica is accordingly expensive. In contrast, the precipitated silica powder in the composition of scar gel of the present disclosure only requires relatively simple processes, and therefore cost of producing it could be significantly lower than that of the fumed silica. For example, sodium silicate and sulfuric acid is mixed to generate a product from the chemical reaction between them, and the product is filtered and classified to obtain the precipitated silica powder in the composition of scar gel of the present disclosure. However, the present disclosure is not limited thereto. In addition, since the method of producing the precipitated silica powder is completely different from that of producing the fumed silica, the precipitated silica powder has higher porosity than that of the fumed silica. In general, the surface area per unit volume of the fumed silica is substantially in a range of 6400-25600 m²/L. However, the precipitated silica powder in the composition of scar gel of the present disclosure is substantially in a range of 9600-57600 m²/L. Therefore, compatibility between the silicone fluid and the volatile diluent (with various degree of polymerization) in the mixture could be further improved because the precipitated silica powder in the composition of scar gel of the present disclosure has higher porosity and surface area per unit volume than those of the fumed silica. Accordingly, the precipitated silica powder in the composition of scar gel of the present disclosure could significantly avoid phase seperation between the silicone fluid and the volatile diluent, and provide long-lasting homogeneous of the mixture of the silicone fluid and the volatile diluent. In other words, when the composition of scar gel of the present disclosure is applied and squeezed from a container, homogeneous gel could be obtained. Therefore, user experience of the composition of scar gel of the present disclosure is better than those using the fumed silica as the thickener. Furthermore, the precipitated silica powder in the composition of scar gel of the present disclosure may be further grinded or shattered to become smaller particles. Therefore, sense of particles or white crumbs issues while applied to skin could be further improved. In addition, the pH value of the fumed silica is substantially in a range of 3.0-4.2. In contrast, the pH value of the precipitated silica powder in the composition of scar gel of the present disclosure could be well controlled in neutral by adjusting the formula or other parameter of the chemical reaction, which produces of the precipitated silica powder. Therefore, the composition of scar gel of the present disclosure is less irritating to skin. In various embodiments of the present disclosure, a pH value of the precipitated silica powder is substantially 6∼8.

It should be also noticed that the composition of scar gel of the present disclosure includes the embellishing powder. The embellishing powder optimizes a color of the composition of scar gel of the present disclosure, and the color is optimized to become similar to that of skin. Therefore, the appearance of the composition of scar gel of the present disclosure could be further enhanced, and it is hard aware that the composition of scar gel of the present disclosure is applied on skin of users. The embellishing powder, for example, may include iron oxide, manganese oxide, chromium oxide, or combinations thereof. However, the present disclosure is not limited thereto. The embellishing powder may include any organic, inorganic pigment powders or mixtures, which have color and do not affect scar recovering. The color of the embellishing powder could be produced by adopting proper materials and various mixture ratios thereof corresponding to various complexions. In summary, the silicone fluid is fully miscible with the volatile diluent, and the precipitated silica powder and the embellishing powder are uniformly dispersed in the silicone fluid and the volatile diluent. In various embodiments of the present disclosure, a weight percentage of the silicone fluid is substantially 60∼74%, a weight percentage of the volatile diluent is substantially 16∼28%, a weight percentage of the precipitated silica powder is substantially 3∼23%, and a weight percentage of the embellishing powder is substantially 0.1∼2%.

Besides, in other various embodiments of the present disclosure, the composition of scar gel of the present disclosure further includes a light-damage protection component, and a weight percentage of the light-damage protection component is substantially 0.1∼10%. The light-damage protection component provides sunscreen function, and therefore the composition of scar gel of the present disclosure could further be sunscreen to avoid pigmentation issue, which is frequently occurred during scar recovering. In various embodiments of the present disclosure, the light-damage protection component includes a physical light-damage protection component, a chemical light-damage protection component, or a combination thereof. The physical light-damage protection component includes titanium dioxide, zinc oxide, zirconium oxide, or combinations thereof. The chemical light-damage protection component includes salicylic acid, avobezone, benzoate-4, cinoxate, dioxybenzone, homosalate, mexoryl SX, methyl anthranilate, octocrylene, oxy-molybdate-40, oxybezone, octyl salicylate, octyl methoxycinnamate, octyl dimethy PABA, padimate-O, para amino benzoic acid, pheny benzimidazole sulfonic acid, roxadimate, sulisobenzone, trolamine salicylate. However, the present disclosure id not limited thereto.

Furthermore, in other various embodiments of the present disclosure, the composition of scar gel of the present disclosure further includes an inorganic salts powder, and a weight percentage of the inorganic salts powder is substantially 0.1∼5%. The inorganic salts powder further enhanced compatibility between the silicone fluid, the volatile diluent, and the precipitated silica powder. Therefore, siloxane in various degree of polymerization within the mixture of the silicone fluid, the volatile diluent, and the precipitated silica powder could be further well mixed without phase seperation, and therefore become more long-lasting homogeneous. The inorganic salts powder comprises barium sulfate, strontium sulfate, magnesium sulfate, ferric hydroxide, calcium hydroxide, sodium chloride, potassium chloride, ammonium chloride, monoethanolamine, diethanolamine chloride, sodium sulfate, sodium phosphate, disodium hydrogen phosphate, sodium tripolyphosphate, magnesium silicate, silicon dioxide, sodium magnesium silicate, sodium silicate, hydrated silica, montmorillonite, lithium magnesium sodium silicate, hectorite, stearyl ammonium montmorillonite, stearyl ammonium hectorite, quaternary ammonium montmorillonite 90, quaternary ammonium montmorillonite 18, quaternary ammonium hectorite 18, or combinations thereof. However, the present disclosure is not limited thereto.

In order to further illustrate the unexpected effect, long-lasting homogeneous, of the composition of scar gel of the present disclosure, the experimental procedure and data of Comparative Examples and Examples are described below:

### Comparative Example 1

The fumed silica (HDK® N20, purchased from Wacker Chemie AG) is mixed with iron oxide, titanium dioxide, and barium sulfate to form a mixture. The mixture is then grinded by a planetary ball milling for 6 hours in rotation speed 360 rpm. After the mixture is grinded, the mixture is added to a mixed liquid including a silicone fluid (Dow Corning® Dimethiconol Blend 20) and a volatile diluent (Dow Corning® Cyclopentasiloxane D5), and is further fully mixed with the mixed liquid to form a gel. Among the gel, the weight percentage of the fumed silica is substantially 9 wt%; the weight percentage of the silicone fluid is substantially 60 wt%; the weight percentage of the volatile diluent is substantially 20 wt%; the weight percentage of the iron oxide is substantially 1 wt%; the weight percentage of the titanium dioxide is substantially 8 wt%; and the weight percentage of the barium sulfate is substantially 2 wt%.

### Comparative Example 2

The composition of materials and procedure of preparing Comparative Example 2 are similar to those of Comparative Example 1. The differences between Comparative Example 2 and Comparative Example 1 are that the weight percentage of the fumed silica is substantially 5wt%, and the weight percentage of the silicone fluid is substantially 64wt%.

### Comparative Example 3

The composition of materials and procedure of preparing Comparative Example 3 are similar to those of Comparative Example 1. The differences between Comparative Example 3 and Comparative Example 1 are that barium sulfate is not included Comparative Example 3, and the weight percentage of the fumed silica is substantially 5wt%, the weight percentage of the silicone fluid is substantially 64wt%, and the weight percentage of the volatile diluent is substantially 22wt%.

### Example 1

The precipitated silica powder (SIPERNAT® 22LS) is mixed with iron oxide, titanium dioxide, and barium sulfate to form a mixture. The mixture is then grinded by a planetary ball milling for 6 hours in rotation speed 360 rpm. After the mixture is grinded, the mixture is added to a mixed liquid including a silicone fluid (Dow Corning® Dimethiconol Blend 20) and a volatile diluent (Dow Corning® Cyclopentasiloxane D5), and is further fully mixed with the mixed liquid to form a composition of scar gel of the present disclosure. Among the composition of scar gel, the weight percentage of the precipitated silica powder is substantially 3 wt%; the weight percentage of the silicone fluid is substantially 66 wt%; the weight percentage of the volatile diluent is substantially 20 wt%; the weight percentage of the iron oxide is substantially 1 wt%; the weight percentage of the titanium dioxide is substantially 8 wt%; and the weight percentage of the barium sulfate is substantially 2 wt%.

### Example 2

The composition of materials and procedure of preparing Example 2 are similar to those of Example 1. The differences between Example 2 and Example 1 are that the weight percentage of the precipitated silica powder is increased to substantially 5wt%, the weight percentage of the silicone fluid is decreased to substantially 64wt%.

### Example 3

The composition of materials and procedure of preparing Example 3 are similar to those of Example 1. The differences between Example 3 and Example 1 are that the weight percentage of the precipitated silica powder is increased to substantially 8wt%, the weight percentage of the silicone fluid is decreased to substantially 61wt%.

### Example 4

The composition of materials and procedure of preparing Example 4 are similar to those of Example 1. The differences between Example 4 and Example 1 are that the weight percentage of the precipitated silica powder is increased to substantially 10wt%, the weight percentage of the silicone fluid is decreased to substantially 59wt%.

### Example 5

The composition of materials and procedure of preparing Example 5 are similar to those of Example 1. The differences between Example 5 and Example 1 are that the weight percentage of the precipitated silica powder is increased to substantially 12wt%, the weight percentage of the silicone fluid is decreased to substantially 57wt%.

### Example 6

The composition of materials and procedure of preparing Example 6 are similar to those of Example 1. The differences between Example 6 and Example 1 are that the weight percentage of the precipitated silica powder is increased to substantially 15wt%, the weight percentage of the silicone fluid is decreased to substantially 54wt%.

### Example 7

The composition of materials and procedure of preparing Example 7 are similar to those of Example 1. The differences between Example 7 and Example 1 are that barium sulfate is not included Example 7, and the weight percentage of the precipitated silica powder is substantially 5wt%; the weight percentage of the silicone fluid is decreased to substantially 64wt%; the weight percentage of the volatile diluent is substantially 22 wt%; the weight percentage of the iron oxide is substantially 1 wt%; and the weight percentage of the titanium dioxide is substantially 8 wt%.

### Example 8

The composition of materials and procedure of preparing Example 8 are similar to those of Example 7. The differences between Example 8 and Example 7 are that barium sulfate is added into the composition of scar gel. The weight percentage of the barium sulfate is substantially 1wt%, and the weight percentage of the volatile diluent is substantially 21wt%.

### Example 9

The composition of materials and procedure of preparing Example 8 are similar to those of Example 7. The differences between Example 8 and Example 7 are that barium sulfate is added into the composition of scar gel. The weight percentage of the barium sulfate is increased to substantially 3wt%, and the weight percentage of the volatile diluent is decreased to substantially 19wt%.

Respectively, 15g of above Comparative Example 1-3 and Examples 1-9 are measured, and respectively filled in three LDPE tubes. Those thirty-six LDPE tubes (each of Comparative Example 1-3 and Examples 1-9 has three tubes to obtain average value in each example) are placed into an oven at 75°C for aging test in 3, 6, 9, and 12 days. Finally, all LDPE tubes are squeezed to respectively collect an amount of upper layer oil, which is caused by phase separation in each example. The experimental data are collected and summarized as following Table 1 and Table 2.

**Table 1: Average amounts of upper layer oil of Comparative Example 1-3 and Example 1-3 after various time**

| Average amounts of upper layer oil (g) | Comparative Example 1 | Comparative Example 2 | Comparative Example 3 | Example 1 | Example 2 | Example 3 |
|---|---|---|---|---|---|---|
| 3 days | 0.03 | 0.02 | 0.03 | 0.00 | 0.00 | 0.00 |
| 6 days | 0.15 | 0.09 | 0.09 | 0.00 | 0.00 | 0.00 |
| 9 days | 0.20 | 0.16 | 0.15 | 0.02 | 0.02 | 0.01 |
| 12 days | 0.29 | 0.26 | 0.24 | 0.10 | 0.02 | 0.01 |

**Table 2: Average amounts of upper layer oil of Example 4-9 after various time**

| Average amounts of upper layer oil (g) | Example 4 | Example 5 | Example 6 | Example 7 | Example 8 | Example 9 |
|---|---|---|---|---|---|---|
| 3 days | 0.00 | 0.00 | 0.00 | 0.00 | 0.00 | 0.00 |
| 6 days | 0.00 | 0.00 | 0.00 | 0.02 | 0.00 | 0.00 |
| 9 days | 0.01 | 0.01 | 0.00 | 0.07 | 0.03 | 0.01 |
| 12 days | 0.02 | 0.02 | 0.01 | 0.13 | 0.04 | 0.01 |

As shown in Table 1 and Table 2, Comparative Examples 1-3 (include the fumed silica as the thickener) respectively generates obvious average amount of upper layer oil in 3 days. In contrast, Examples 1-9 (include the precipitated silica powder as the thickener) of the present disclosure respectively has longer-lasting homogenous effect, and generates some average amount of upper layer oil in 9 days. According to data of Example 1-9, with increased weight percentage weight of the precipitated silica powder, homogenous system of the composition of scar gel could be significantly extended to 12 days (as Example 2-6). It can be concluded that the precipitated silica powder has better capability of keeping long-lasting homogenous system of the composition of scar gel better than the fumed silica does. Compatibility between the silicone fluid and the volatile diluent (with various degree of polymerization) could be further improved by the precipitated silica powder in the composition of scar gel of the present disclosure, and therefore the composition of scar gel of the present disclosure could be a relatively long-lasting homogenous system. Accordingly, excellent use of perception could be offered. In addition, according to data of Example 4 and 7-9, it can be concluded that the inorganic salts powder could also significantly increase compatibility between the silicone fluid and the volatile diluent, and therefore the composition of scar gel of the present disclosure could be the long-lasting homogenous system for a longer period. As shown in data of Example 7 (without the inorganic salts powder), obvious average amount of upper layer oil is observed in 9 days. In contrast, as shown in data of Example 4 and 8-9 (with the inorganic salts powder), no or subtle average amount of upper layer oil is observed.

It should be noticed that the composition of scar gel according various embodiments of the present disclosure includes the precipitated silica powder as the thickener instead of the fumed silica. Therefore, the processes for producing the composition of scar gel could be simplified, and the cost of producing the composition of scar gel could be significantly reduced. Furthermore, compatibility between the silicone fluid and the volatile diluent (with various degree of polymerization) could be significantly improved by the precipitated silica powder in the composition of scar gel of the present disclosure, and therefore the composition of scar gel of the present disclosure could be a relatively long-lasting homogenous system. Accordingly, excellent use of perception could be offered. On the other hand, the embellishing powder in the composition of scar gel of the present disclosure further enhances appearance during applying the scar gel. In addition, the inorganic salts or the light-damage protection component could be optionally added in the composition of scar gel of the present disclosure. The inorganic salts in the composition of scar gel of the present disclosure could also significantly increase compatibility between the silicone fluid and the volatile diluent, and therefore the composition of scar gel of the present disclosure could be the long-lasting homogenous system for a longer period. The light-damage protection component in the composition of scar gel of the present disclosure provides sunscreen function, and therefore the composition of scar gel of the present disclosure could further be sunscreen to avoid pigmentation issue, which is frequently occurred during scar recovering.

## Claims

1. A composition of scar gel, comprising:
a silicone fluid;
a volatile diluent;
a precipitated silica powder; and
an embellishing powder,
wherein the silicone fluid is fully miscible with the volatile diluent, and the precipitated silica powder and the embellishing powder are uniformly dispersed in the silicone fluid and the volatile diluent.

2. The composition of scar gel of claim 1, wherein the embellishing powder comprises iron oxide, manganese oxide, chromium oxide, or combinations thereof.

3. The composition of scar gel of claim 1, wherein a weight percentage of the silicone fluid is substantially 60∼74%, a weight percentage of the volatile diluent is substantially 16∼28%, a weight percentage of the precipitated silica powder is substantially 3∼23%, and a weight percentage of the embellishing powder is substantially 0.1∼2%.

4. The composition of scar gel of claim 1, wherein a pH value of the precipitated silica powder is substantially 6∼8.

5. The composition of scar gel of claim 1, further comprising a light-damage protection component, and a weight percentage of the light-damage protection component is substantially 0.1∼10%.

6. The composition of scar gel of claim 5, wherein the light-damage protection component comprises a physical light-damage protection component, a chemical light-damage protection component, or a combination thereof.

7. The composition of scar gel of claim 6, wherein the physical light-damage protection component comprises titanium dioxide, zinc oxide, zirconium oxide, and the chemical light-damage protection component comprises salicylic acid, avobezone, benzoate-4, cinoxate, dioxybenzone, homosalate, mexoryl SX, methyl anthranilate, octocrylene, oxy-molybdate-40, oxybezone, octyl salicylate, octyl methoxycinnamate, octyl dimethy PABA, padimate-O, para amino benzoic acid, pheny benzimidazole sulfonic acid, roxadimate, sulisobenzone, trolamine salicylate.

8. The composition of scar gel of claim 1, further comprising an inorganic salts powder, and a weight percentage of the inorganic salts powder is substantially 0.1∼5%.

9. The composition of scar gel of claim 8, wherein the inorganic salts powder comprises barium sulfate, strontium sulfate, magnesium sulfate, ferric hydroxide, calcium hydroxide, sodium chloride, potassium chloride, ammonium chloride, monoethanolamine, diethanolamine chloride, sodium sulfate, sodium phosphate, disodium hydrogen phosphate, sodium tripolyphosphate, magnesium silicate, silicon dioxide, sodium magnesium silicate, sodium silicate, hydrated silica, montmorillonite, lithium magnesium sodium silicate, hectorite, stearyl ammonium montmorillonite, stearyl ammonium hectorite, quaternary ammonium montmorillonite 90, quaternary ammonium montmorillonite 18, quaternary ammonium hectorite 18, or combinations thereof.

10. The composition of scar gel of claim 1, wherein the silicone fluid comprises a mixture of polydimethylsiloxane and dimethylsiloxane, and the volatile diluent comprises linear or cyclic volatile siloxane.

## Patentansprüche

1. Narbengelzusammensetzung, umfassend:
ein Silikonfluid;
ein flüchtiges Verdünnungsmittel;
ein Fällungskieselsäurepulver und
ein Verschönerungspulver,
wobei das Silikonfluid vollständig mit dem flüchtigen Verdünnungsmittel mischbar ist und das Fällungskieselsäurepulver und das Verschönerungspulver einheitlich in dem Silikonfluid und dem flüchtigen Verdünnungsmittel dispergiert sind.

2. Narbengelzusammensetzung nach Anspruch 1, wobei das Verschönerungspulver Eisenoxid, Manganoxid, Chromoxid oder Kombinationen davon umfasst.

3. Narbengelzusammensetzung nach Anspruch 1, wobei der Gewichtsprozentanteil des Silikonfluids im Wesentlichen 60-74% beträgt, der Gewichtsprozentanteil des flüchtigen Verdünnungsmittels im Wesentlichen 16-28% beträgt, der Gewichtsprozentanteil des Fällungskieselsäurepulvers im Wesentlichen 3-23% beträgt und der Gewichtsprozentanteil des Verschönerungspulvers im Wesentlichen 0,1-2% beträgt.

4. Narbengelzusammensetzung nach Anspruch 1, wobei der pH-Wert des Fällungskieselsäurepulvers im Wesentlichen 6-8 beträgt.

5. Narbengelzusammensetzung nach Anspruch 1, ferner umfassend eine Lichtschädigungsschutzkomponente, wobei der Gewichtsprozentanteil der Lichtschädigungsschutzkomponente im Wesentlichen 0,1-10% beträgt.

6. Narbengelzusammensetzung nach Anspruch 5, wobei die Lichtschädigungsschutzkomponente eine physikalische Lichtschädigungsschutzkomponente, eine chemische Lichtschädigungsschutzkomponente oder eine Kombination davon umfasst.

7. Narbengelzusammensetzung nach Anspruch 6, wobei die physikalische Lichtschädigungsschutzkomponente Titandioxid, Zinkoxid, Zirkoniumoxid umfasst und die chemische Lichtschädigungsschutzkomponente Salicylsäure, Avobenzon, Benzoat-4, Cinoxat, Dioxybenzon, Homosalat, Mexoryl SX, Methylanthranilat, Octocrylen, Oxymolybdat-40, Oxybenzon, Octylsalicylat, Octylmethoxycinnamat, Octyl-dimethyl-PABA, Padimat-O, para-Aminobenzoesäure, Phenylbenzimidazolsulfonsäure, Roxadimat, Sulisobenzon, Trolaminsalicylat umfasst.

8. Narbengelzusammensetzung nach Anspruch 1, ferner umfassend ein Pulver anorganischer Salze, wobei der Gewichtsprozentanteil des Pulvers anorganischer Salze im Wesentlichen 0,1-5% beträgt.

9. Narbengelzusammensetzung nach Anspruch 8, wobei das Pulver anorganischer Salze Bariumsulfat, Strontiumsulfat, Magnesiumsulfat, Eisen(III)-hydroxid, Calciumhydroxid, Natriumchlorid, Kaliumchlorid, Ammoniumchlorid, Monoethanolamin, Diethanolaminchlorid, Natriumsulfat, Natriumphosphat, Dinatriumhydrogenphosphat, Natriumtripolyphosphat, Magnesiumsilicat, Siliciumdioxid, Natriummagnesiumsilicat, Natriumsilicat, Siliciumdioxidhydrat, Montmorillonit, Lithiummagnesiumnatriumsilicat, Hectorit, Stearylammoniummontmorillonit, Stearylammoniumhectorit, quat.-Ammoniummontmorillonit 90, quat.-Ammoniummontmorillonit 18, quat.-Ammoniumhectorit 18 oder Kombinationen davon umfasst.

10. Narbengelzusammensetzung nach Anspruch 1, wobei das Silikonfluid eine Mischung von Polydimethylsiloxan und Dimethylsiloxan umfasst und das flüchtige Verdünnungsmittel lineares oder cyclisches flüchtiges Siloxan umfasst.

## Revendications

1. Composition de gel cicatrisant, comprenant :
un fluide siliconé ;
un diluant volatil ;
une poudre de silice précipitée ; et
une poudre embellissante,
où le fluide siliconé est entièrement miscible au diluant volatil, et la poudre de silice précipitée et la poudre embellissante sont uniformément dispersées dans le fluide siliconé et le diluant volatil.

2. Composition de gel cicatrisant selon la revendication 1, où la poudre embellissante comprend de l'oxyde de fer, de l'oxyde de manganèse, de l'oxyde de chrome ou leurs combinaisons.

3. Composition de gel cicatrisant selon la revendication 1, où le pourcentage massique du fluide siliconé est substantiellement d'environ 60 à 74 %, le pourcentage massique du diluant volatil est substantiellement d'environ 16 à 28 %, le pourcentage massique de la poudre de silice précipitée est substantiellement d'environ 3 à 23 %, et le pourcentage massique de la poudre embellissante est substantiellement d'environ 0,1 à 2 %.

4. Composition de gel cicatrisant selon la revendication 1, où la valeur du pH de la poudre de silice précipitée est substantiellement d'environ 6 à 8.

5. Composition de gel cicatrisant selon la revendication 1, comprenant en outre un composant de protection contre les dommages provoqués par la lumière, et le pourcentage massique du composant de protection contre les dommages provoqués par la lumière est substantiellement d'environ 0,1 à 10 %.

6. Composition de gel cicatrisant selon la revendication 5, où le composant de protection contre les dommages provoqués par la lumière comprend un composant de protection physique contre les dommages provoqués par la lumière, un composant de protection chimique contre les dommages provoqués par la lumière, ou l'une de leurs combinaisons.

7. Composition de gel cicatrisant selon la revendication 6, où le composant de protection physique contre les dommages provoqués par la lumière comprend les suivants : dioxyde de titane, oxyde de zinc, oxyde de zirconium, et le composant de protection chimique contre les dommages provoqués par la lumière comprend les suivants : acide salicylique, avobézone, benzoate-4, cinoxate, dioxybenzone, homosalate, méxoryl SX, anthranilate de méthyle, octocrylène, oxy-molybdate-40, oxybézone, salicylate d'octyle, méthoxycinnamate d'octyle, octyl-diméthyl-PABA, padimate-O, acide para-amino-benzoïque, acide phénybenzimidazolesulfonique, roxadimate, sulisobenzone, trolamine salicylate.

8. Composition de gel cicatrisant selon la revendication 1, comprenant en outre une poudre de sels inorganiques, et le pourcentage massique de la poudre de sels inorganiques est substantiellement d'environ 0,1 à 5 % en masse.

9. Composition de gel cicatrisant selon la revendication 8, où la poudre de sels inorganiques comprend les suivants : sulfate de baryum, sulfate de strontium, sulfate de magnésium, hydroxyde ferrique, hydroxyde de calcium, chlorure de sodium, chlorure de potassium, chlorure d'ammonium, monoéthanolamine, chlorure de diéthanolamine, sulfate de sodium, phosphate de sodium, hydrogénophosphate de disodium, tripolyphosphate de sodium, silicate de magnésium, dioxyde de silicium, silicate de sodium et de magnésium, silicate de sodium, silice hydratée, montmorillonite, silicate de lithium, de magnésium et de sodium, hectorite, montmorillonite de stéarylammonium, hectorite de stéarylammonium, montmorillonite d'ammonium quaternaire 90, montmorillonite d'ammonium quaternaire 18, hectorite d'ammonium quaternaire 18, ou leurs combinaisons.

10. Composition de gel cicatrisant selon la revendication 1, où le fluide siliconé comprend un mélange de polydiméthylsiloxane et de diméthylsiloxane, et le diluant volatil comprend un siloxane volatil linéaire ou cyclique.
